# EUROPEAN PATENT SPECIFICATION

(11) **EP 1 322 748 B1**
(45) Date of publication and mention of the grant of the patent: **02.12.2009**
(21) Application number: 01972471.5
(22) Date of filing: 19.09.2001
(51) Int. Cl.: C12N 5/08, A61K 31/122, A61K 35/12, A61P 25/00, C07C 50/28

(54) **STEM CELL DIFFERENTIATION-INDUCING PROMOTER**
STEMZELLDIFFERENZIERUNG INDUZIERENDE PROMOTER
PROMOTEUR INDUISANT LA DIFFERENTIATION DE CELLULES SOUCHES

(30) Priority: 04.10.2000 JP 2000304746
(43) Date of publication of application: 02.07.2003
(73) Proprietor: Meiji Dairies Corporation, Koto-ku, Tokyo 136-8908 (JP)
(72) Inventor: LUU, Bang, Chimie Subst. Nat., ULP-CNRS UMR 7123, 67070 Strasbourg Cedex (FR); MOHIER, Eliane, Chimie S. Nat., ULP-CNRS UMR 7123, 67070 Strasbourg Cedex (FR); YAMADA, Masashi, c/o Meiji Dairies Corp., Koto-ku, Tokyo 136-8908 (JP); SUMA, Yukie, c/o Meiji Dairies Corp., Koto-ku, Tokyo 136-8908 (JP); SUZUKI, Hiroto, c/o Meiji Dairies Corp., Koto-ku, Tokyo 136-8908 (JP)
(74) Representative: Wächtershäuser, Günter
(86) International application number: PCT/JP2001/008136
(87) International publication number: WO 2002/029014

(56) References cited:
- WO-A-99/08987
- GIRLANDA-JUNGES C ET AL: "Effect of Cyclohexenonic Long Chain Fatty Alcohols on Neurite Outgrowth. Study on Structure-Activity Relationship" TETRAHEDRON, ELSEVIER SCIENCE PUBLISHERS, AMSTERDAM, NL, vol. 54, no. 27, 2 July 1998 (1998-07-02), pages 7735-7748, XP004122484 ISSN: 0040-4020
- BJÖRKLUND A. ET AL,: 'Cell replacement therapies for central nervous system disorders.' NATURE NEUROSCIENCE vol. 3, no. 6, 2000, page 537

## Description

### Technical Field:

The present invention relates to the use of a stem-cell differentiation-inducing promoter capable of promoting differentiation-induction of stem cells.

### Background Art:

Nervous disorders such as Alzheimer's disease and Pick's disease having a main lesion at the cerebral cortex, Parkinson disease and Huntington chorea having a main lesion at the cerebral basal nuclei, spino-cerebellar degeneration having a main lesion at the cerebellum, and amyotrophic lateral sclerosis having a main lesion at the spinal cord; or bone diseases such as osteoporosis and fracture are presumed to be caused by the dysfunction of tissues or organs due to degeneration or reduction, or even death of neurocytes or osteoblasts.

Stem cells typified by mallow stem cells, nerve stem cells and epidermic stem cells are undifferentiated. When some of cells die in a programmed death way, stem cells start differentiation so as to compensate for the lost cells, thereby largely contributing to the keep-up of biological functions. These stem cells are available from ES cells. ES cells have a capacity of differentiating into any cell constituting organs or tissues.

In recent years, an attempt has been made to transplant ES cells or stem cells into the tissue that has lost its function and allow them to differentiate into a desired cell expressing a specific biological function, thereby ameliorating or treating the corresponding morbid state. There is accordingly a demand for the development of a substance promoting differentiation-induction of ES cells or stem cells.

It has been known that during differentiation of stem cells into neurocytes or osteoblasts, there exist differentiation promoting factors such as bone morphogenetic protein (BMP), brain derived neurotrophic factor (BDNF) and basic fibroblast growth factor (bFGF) (see e.g. Reynolds B.A. and Weiss S., 1996. Clonal and Population Analyses Demonstrate that an EGF-Responsive Mammalian Embryonic CNS Precursor is a Stem Cell. Developmental Biology, 175:1-13 and Weiss S. et al. 1996. Multipotent CNS Stem Cells are Present in the Adult Mammalian Spinal Cord and Ventricular Neuroaxis. The Journal of Neuroscience, 16:7599-7609.) Therefore, it can be considered that such differentiation inducing factor can be used for prevention or treatment of the above-described diseases. Such factors, however, are peptides having a large molecular weight and thus are easily cleaved in vivo and cannot cross the blood brain barrier, thus markedly limiting their administration method.

There is accordingly a demand for the development of a synthetic compound which promotes differentiation-induction of stem cells into mature cells and has a molecular weight low enough to permit easy handling. WO99/08987 discloses cyclohexenone long-chain alcohols and medicaments containing the same. Further effects of cyclohexenone long-chain fatty alcohols are known from Tetrahedron 54(1998)7735-7748.

### Disclosure of the Invention

An object of the present invention is to provide the use of a low-molecular weight substance which promotes differentiation-induction of stem cells and is useful for the prevention and therapy of diseases, such as bone diseases or neurogenic diseases, resulting from degeneration or reduction or even death of cells.

In view of the foregoing, the present inventors have studied various low-molecular weight compounds capable of promoting differentiation of stem cells into cells expressing a specific biological function. As a result, it has been found that a long-chain alcohol having a cyclohexenone skeleton represented by the formula (1) shown below has an excellent action for promoting differentiation -induction of stem cells to complete the present invention.

According to the present invention, there is provided the use of a cyclohexenone long-chain alcoholic derivative represented by the following formula (1): 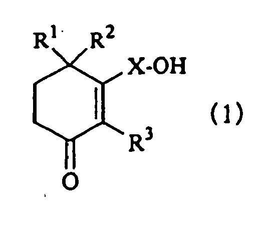 [wherein, R¹, R² and R³ each independently represents a hydrogen atom or a methyl group and X represents a linear or branched C₁₀₋₁₈ alkylene or alkenylene group as further defined by the claim.

The cyclohexenone long-chain alcoholic derivative used according to the present invention promotes differentiation -induction of stem cells into cells which express a specific biological function. Thus, a medicament comprising the derivative is useful as a preventive or remedial drug for diseases caused by degeneration or reduction of various tissues or cells, or by cell death, for example, nervous diseases such as Alzheimer's disease, Pick's disease, Parkinson disease, Huntington chorea, spino-cerebellar degeneration and amyotrophic lateral sclerosis; bone diseases such as osteoporosis and fracture; circulatory diseases such as angina pectoris, retinopathy, arteritis obliterans, and myopathy such as muscular dystrophy and congenital myopathy.

There is also disclosed a method for treating diseases caused by degeneration or reduction of tissues or cells or by cell death, which comprises transplanting stem cells into the part of the tissues of a patient that has lost its specific biological function, administering to the patient an effective amount of the cyclohexenone derivative of the formula (1), pharmaceutically acceptable salt, solvate or hydrate thereof to promote differentiation-induction of the stem cells in the patient into cells expressing the specific biological function. Alternatively, stem cells in vitro may be differentiated into cells expressing the specific biological function by adding thereto an effective amount of the cyclohexenone derivative of the formula (1), pharmaceutically acceptable salt, solvate or hydrate thereof, and then the resulting cells expressing the specific biological function may be transplanted into the patient in need thereof.

### Detailed Description of the Preferred Embodiments

The stem-cell differentiation-inducing promoter according to the present invention means a substance capable of promoting differentiation-induction of a nerve stem cell into a cell expressing a specific biological function.

The term "stem cell" as used herein means an undifferentiated cell having a capacity of differentiating into a cell expressing a specific biological function, and embraces undifferentiated precursor cells which, although not identified morphologically, have already been oriented to differentiation into a specific organ or tissue.

Although no particular limitation is imposed on the cells expressing a specific biological function, examples thereof include osteocyte, neurocyte, blood vessel and muscle, with neurocyte being preferred.

In the cyclohexenone long-chain alcoholic derivative represented by the formula (I), X represents a linear or branched C₁₀₋₂₈ alkylene and alkenylene group. The branched alkylene or alkenylene group may contain as a side chain a C₁₋₁₀ alkyl group. Examples of the alkyl group as the side chain include methyl, ethyl, propyl, isopropyl, butyl, isobutyl, sec-butyl, tert-butyl, pentyl, isopentyl, neopentyl, tert-pentyl, hexyl, isohexyl, heptyl, octyl, nonyl and decyl groups. Among them, methyl group is particularly preferred.

The side chain is preferably substituted at the 3-and/or 7-position of the linear alkylene or alkenylene group (which has an alkene structure having at least one carbon-carbon double bond). As the X, a linear C₁₀₋₂₈ alkylene group is preferred, with a linear C₁₀₋₁₈ alkylene group being particularly preferred.

R¹, R² and R³ each independently represents a hydrogen atom or a methyl group, and preferably at least one of R¹, R² and R³ represents a methyl group.

The compound represented by the formula (1) may be in the form of a pharmaceutically acceptable salt, a solvate or a hydrate thereof. The compound (1) has various isomers and these isomers are also embraced by the present invention.

The cyclohexenone long-chain alcoholic derivative represented by the formula (1) can be prepared, for example, in accordance with the following Process A or Process B. 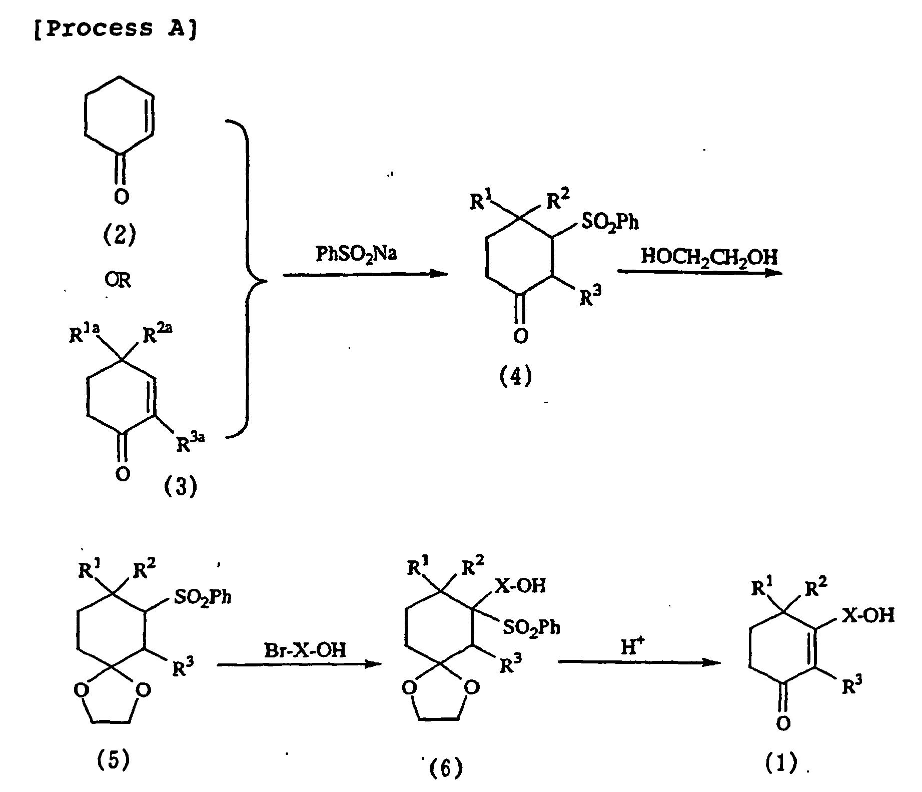 [wherein, R^{1a}, R^{2a} and R^{3a} each independently represents a hydrogen atom or a methyl group with the proviso that at least one of R^{1a}, R^{2a} and R^{3a} represents a methyl group, Ph represents a phenyl group, and X, R¹, R² and R³ have the same meanings as described above].

Specifically, the compound (1) can be prepared by reacting cyclohexenone (2) or a methyl-substituted-2-cyclohexen-1-one (3) with a phenylsulfinic acid salt in the presence of an acid, reacting the resulting compound (4) with ethylene glycol, reacting the resulting ketal derivative (5) with a .-halogenoalkanol or .-halogenoalkenol, and subjecting the resulting compound (6) to an acid treatment to eliminate the protecting group.

The methyl-substituted 2-cyclohexen-1-one (3) used herein as a raw material can be obtained by reacting a methyl-substituted cyclohexanone with a trialkylsilyl halide in the presence of butyl lithium, followed by oxidation in the presence of a palladium catalyst.

The reaction of cyclohexenone (2) or a methyl-substituted-2-cyclohexen-1-one (3) with a phenylsulfinic acid salt such as sodium phenylsulfinate is preferably effected in the presence of an acid such as hydrochloric acid, sulfuric acid or phosphoric acid at a temperature in a range of from 0 to 100°C for 5 to 40 hours.

As the .-halogenoalkanol to be reacted with the ketal derivative (5), a .-bromoalkanol is preferably used. It is desired that the ketal derivative (5) is reacted with a .-halogenoalkanol in the presence of a metal compound such as butyl lithium under low temperature conditions.

Elimination of the phenylsulfonyl group and ketal-protecting groups from the compound (6) is preferably effected by reacting the compound (6) with an acid such as paratoluenesulfonic acid. 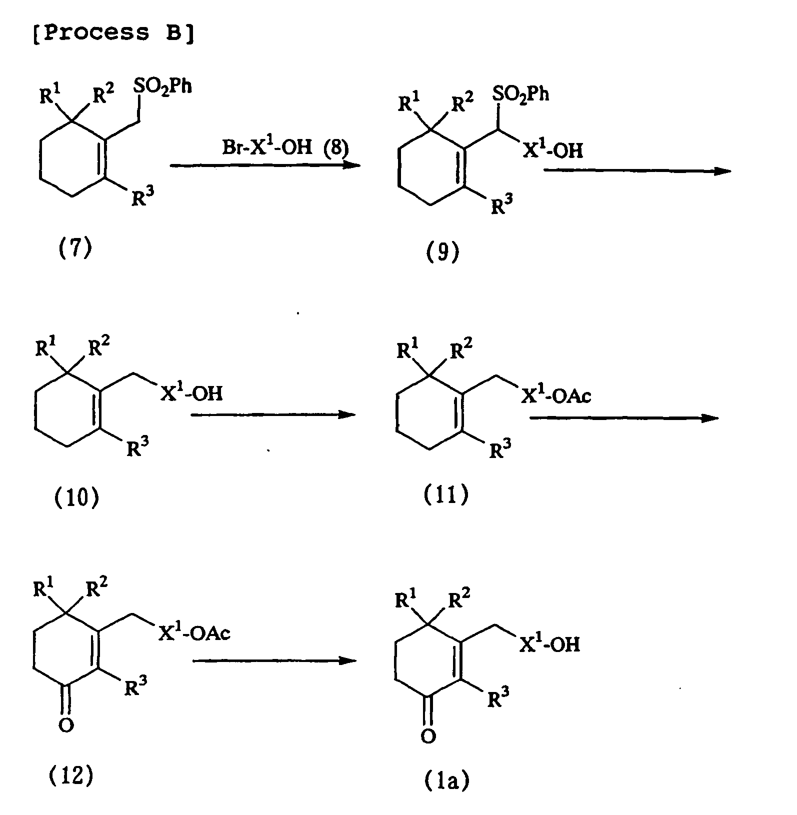 [wherein, X¹ represents a C₉₋₂₇ alkylene or alkenylene group, Ac represents an acyl group, and R¹, R², R³ and Ph have the same meanings as described above].

Specifically, the compound (1a) can be obtained by reacting the compound (7) [prepared in accordance with the process described in, for example, Synthesis, Nov., (1996)] with a .-bromoalcohol (8), eliminating the phenylsulfonyl group from the resulting compound (9), protecting the hydroxy group of the resulting compound (10), oxidizing the resulting compound (11), and then eliminating the hydroxy-protecting group from the resulting compound (12).

The reaction of the compound (7) with the . - bromoalcohol (8) is preferably conducted in the presence of a metal compound such as butyl lithium under low temperature conditions.

The phenylsulfonyl group is eliminated from the compound (9) by reacting the compound (9) with a phosphate salt or the like in the presence of, for example, sodium amalgam.

As the hydroxy-protecting group of the compound (10), an acetyl group is preferred. The compound (10) is protected, for example, by reacting it with acetic anhydride.

The compound (11) is oxidized by reacting it with an alkyl hydroperoxide such as t-butyl hydroperoxide in the presence of a metal compound such as ruthenium trichloride.

The deprotection of the compound (12) is preferably conducted by hydrolyzing it in the presence of a base such as potassium carbonate.

The cyclohexenone long-chain alcoholic derivative (1) of the present invention thus obtained favors differentiation and induction of nerve stem cells into neurocytes as will be described later in Test 1. Accordingly, a medicament containing the derivative (1) is useful as a preventive or remedial drug for diseases caused by degeneration or reduction of various tissues or cells, or cell death.

The cyclohexenone long-chain alcoholic derivative (1) of the present invention is a low-molecular weight compound and thus can be administered either orally as an oral preparation or by parenteral (intramuscular, subcutaneous, intravenous, suppository, or the like) administration.

The oral preparations can be formulated into tablets, covered or coated tablets, granules, capsules, solutions, syrups, elixirs, oil or aqueous suspensions in a manner known per se in the art after addition of an excipient and, if necessary, a binder, a disintegrator, a lubricant, a colorant and/or a corrigent.

Examples of the excipient include lactose, corn starch, sucrose, glucose, sorbitol and crystalline cellulose. Examples of the binder include polyvinyl alcohol, polyvinyl ether, ethyl cellulose, methyl cellulose, gum arabic, tragacanth, gelatin, shellac, hydroxypropyl cellulose, hydroxypropyl starch and polyvinyl pyrrolidone.

Examples of the disintegrator include starch, agar, gelatin powder, crystalline cellulose, calcium carbonate, sodium bicarbonate, calcium citrate, dextran and pectin. Examples of the lubricant include magnesium stearate, talc, polyethylene glycol, silica and hardened vegetable oil. As the colorant, those which are pharmaceutically acceptable as an additive can be used. Examples of the corrigent include cocoa powder, menthol, aromatic acid, peppermint oil, camphor and cinnamon powder.

The tablets and granules may be coated with sugar, gelatin or the like, as needed.

Injections such as subcutaneous, intramuscular or intravenous injections are formulated in a manner known per se in the art by adding a pH regulator, buffer, stabilizer and/or preservative as needed. It is also possible to place the injection solution in a vial or the like and lyophilize the solution into a solid preparation which can be reconstituted immediately before use. One dose may be placed in a vial or alternatively, multiple doses may be placed in one vial.

For a human adult, the dose of the compound of the invention as a medicament is usually within a range of from 0.01 to 1000 mg/day, with a range of from 0.1 to 100 mg/day being preferred. This daily dose is administered once a day or may be divided into 2 to 4 portions.

### Examples

The present invention will be hereinafter described in further detail by way of examples.

### Preparation Example 1

(1) To a 20 ml THF solution of 7 ml of N,N-diisopropylamine, 35.4 ml of a 1.4M n-butyl lithium solution was added dropwise at -78°C, followed by stirring at 0°C for 30 minutes. The resulting diisopropylamino lithium (LDA) solution was then added dropwise to a 10 ml THF solution of 4 ml of 4-methylcyclohexan-1-one at -78°C. After stirring at -78°C for 1 hour, 6.5 ml of trimethylsilyl chloride was added dropwise. After stirring at room temperature for 1 hour, the reaction mixture was poured into an aqueous sodium bicarbonate solution. The resulting mixture was extracted with diethyl ether. The organic layer was washed with saturated saline, dried over magnesium sulfate and distilled under reduced pressure to remove the solvent, whereby 5.83 g of 4-methyl-1-(trimethylsilyloxy)-1-cyclohexene was obtained (yield: 96%).
4-Methyl-1-(trimethylsilyloxy)-1-cyclohexene
Molecular weight: 184 (C₁₀H₂₀OSi)
TLC: (hexane:ethyl acetate = 8:2) Rf=0.8
¹H-NMR (200MHz, CDCl₃). : 0.17(s,9H,Si-(CH₃)₃), 0.94(d,J=6.2Hz,3H,H-7), 1.2-1.43 (m, 1H,H-4), 1.57-1.76(m,3H,H-3,6), 1.88-2.14(m,3H,H-5), 4.8-4.83(m, 1H,H-2). ¹³C-NMR (50MHz, CDCl₃). : 0.3 (Si-(CH₃)₃), 21.2(C-7), 28.3(C-4), 29.6(C-5), 31.3(C-6), 32.3(C-3), 103.5(C-2), 150.1(C-1).
IR(NaCl): 3052, 3021, 2954, 2926, 1670, 1457, 1371, 1252, 1190, 1046, 892, 844.
(2) A catalytic amount of palladium acetate was added to a 70 ml dimethylsulfoxide (DMSO) solution of 3.53 g of 4-methyl-1-(trimethylsilyloxy)-1-cyclohexene, followed by stirring while introducing oxygen for 6 hours. After the addition of water at 0°C, the reaction mixture was filtered and then extracted with ethyl ether. The solvent was distilled off from the organic layer under reduced pressure and the residue was dissolved in hexane-water. The resulting solution was extracted with hexane. The hexane layer was washed with saturated saline and dried over magnesium sulfate. The solvent was distilled off under reduced pressure, whereby 4-methyl-2-cyclohexen-1-one was obtained in the form of oil (Yield: 72%).
4-Methyl-2-cyclohexen-1-one
Molecular weight: 110 (C₇H₁₀O)
TLC: (hexane:AcOEt = 8:2) Rf=0.35
¹H-NMR (200MHz, CDCl₃). : 1.15 (d,J=7.1Hz,3H,H-7), 1.56-1.76 (m, 1H, H-5a), 2.1 (dqa, J_{gem}=13.3Hz, ³J=4.9Hz, 1H,H-5e), 2.26-2.48(m,2H,H-6), 2.49-2.62(m, 1H, H-4), 5.94 (dd, ³J=10.1Hz,⁴J=2.5Hz,1H,H-2), 6.79 (ddd,³J=10.1Hz, ³J=2.7Hz,⁴J=1.5Hz,1H,H-3).
¹³C-NMR (50MHz, CDCl₃). : 20.1 (C-7), 29.6(C-5), 30.9(C-4), 36.8(C-6), 128.4(C-2), 156.2 (C-3), 199.7(C-1).
IR(NaCl): 3025, 2958, 2932, 1683, 1617, 1458, 1391, 1375, 1251, 1094, 1053, 1016, 828, 750.
(3) Benzenesulfinic acid sodium salt (3.0 g) was added to a solution containing 1.52 g of 4-methyl-2-cyclohexen-1-one and 9 ml of water. 1N Hydrochloric acid (18 ml) was added dropwise to the resulting solution. After stirring at room temperature for 24 hours, the crystals so precipitated were filtered and washed with water, isopropanol and cold diethyl ether. After recrystallization from isopropanol, 4-methyl-3-(phenylsulfonyl)-cyclohexan-1-one was obtained in the form of white crystals (yield: 72%).
4-Methyl-3-(phenylsulfonyl)-cyclohexan-1-one
Molecular weight: 252 (C₁₃H₁₆O₃S)
Melting point: 71 to 74°C
TLC: (hexane:ethyl acetate = 6:4) Rf=0.2
¹H-NMR (200MHz, CDCl₃),
-trans .: 1.32(d,J=6.9Hz,3H,H-7), 1.5-1.7 (m, 1H, H-5), 2.15-2.3 (m, 1H, H-5), 2.35-2.5(m,3H,H-4,6), 2.55-2.68(m,2H,H-2), 3.17 (ddd, J=8Hz, J=6.6Hz, J=6.4Hz,1H,H-3), 7.52-7.72(m,3H,H ar.-3',4'), 7.83-7.9(m,2H,H ar.-2'),
-cis .: 1.44 (d, J=7.1Hz, 3H, H-7), 1.75-1.9 (m, 1H, H-5), 1.95-2.1(m, 1H, H-5), 2.23-2.5(m,3H,H-4,6), 2.73-2.9(m,2H,H-2), 3.34 (dt, J=12.9Hz, J=4Hz, 1H, H-3), 7.52-7.72(m,3H,H ar.-3',4'), 7.83-7.9(m,2H,H ar.-2').
¹³C-NMR (50MHz, CDCl₃)
-trans .: 20.3(C-7), 28.5(C-4), 30.4(C-5), 37.9(C-6 or -2), 38.6(C-2 or -6), 66.3(C-3), 128.6(C ar.-2' or -3'), 129.1 (C ar.-3' or -2'), 133.9 (C ar.-4'), 137.2 (C ar.-1'), 206.6(C-1).
-cis .: 13 (C-7), 27.2(C-4), 31.1(C-5), 35.9(C-6 or -2), 36.9(C-2 or -6), 64.6(C-3), 128.3(C ar.-2' or-3'), 129.1(C ar.-3' or -2'), 133.9(C ar.-4'), 138(C ar.-1'), 206.6 (C-1). MS(EI): 111.1 (M-SO₂Ph,88) 110.1(27), 83.15(32), 77.1 (29), 69.1(36), 55.2(100).
(4) To a solution of 2.45 g of 4-methyl-3-(phenylsulfonyl)-cyclohexan-1-one in 40 ml of benzene, were added 0.7 ml of 1,2-ethanediol and 0.2 g of paratoluenesulfonic anhydride. The resulting mixture was heated under reflux for 4 hours. After the reaction, a 2M aqueous sodium bicarbonate solution was added and the resulting mixture was extracted with ethyl acetate three times. The combined organic layers were washed with saturated saline, and dried over magnesium sulfate. The solvent was then distilled off under reduced pressure. The residue was recrystallized from diethyl ether, whereby 1,1-(ethylenedioxy)-4-methyl-3-(phenylsulfonyl)-cyclohexane was obtained in the form of white crystals (yield: 97%).
1,1-Ethylenedioxy-4-methyl-3-phenylsulfonyl-cyclohexane Molecular weight: 296 (C₁₅H₂₀O₄S)
Melting point: 105 to 106°C
TLC: (hexane:ethyl acetate = 6:4) Rf=0.3
¹H-NMR (200 MHz, CDCl₃),
-trans .: 1.23(d,J=6.1Hz,3H,H-7), 1.37-1.77 (m,6H,H-2a,4,5,6), 1.84 (ddd, J_{gem}=12.9Hz, ³J=3.7Hz, ⁴J=2.7Hz, 1H, H-2e), 3.02 (ddd, ³J=13Hz, ³J=10.3Hz, ³J=3.7Hz, 1H, H-3), 3.71-3.91 (m, 4H, O-CH₂-CH₂-O), 7.48-7.67(m,3H,H ar.-3',4'), 7.8-7.88(m,2H,H ar.-2')
-cis .: 1.18(d,J=6.9Hz,3H,H-7), 1.37-1.77(m,4H,H-5,6), 1.84 (ddd, J_{gem}= 13Hz, ³J=3.7Hz, ⁴J=2.7Hz, 1H, H-2e), 2.02(t,J=13Hz,1H,H-2a), 2.30-2.45 (m, 1H, H-4), 3.29 (dt, ³J=13Hz, ³J=3.7Hz, 1H, H-3), 3.71-3.91 (m, 4H, O-CH₂-CH₂-O), 7.48-7.67(m,3H,H ar.-3',4'), 7.8-7.88(m,2H,H ar.-2').
¹³C-NMR (50MHz, CDCl₃)
-trans .: 20.4(C-7), 31.9(C-4), 32.6(C-5), 34.1(C-6), 35.8(C-2), 64.4(CH₂-O), 66.8(C-3), 107.9(C-1), 128.6(C ar.-3' or -2'), 129 (C ar.-2' or -3'), 133.5(C ar.-4'), 138(C ar.-1').
IR(KBr): 3060, 2968, 2938, 1583, 1448, 1301, 1267, 1158, 1144, 1082, 1023, 939, 916, 838, 749, 718, 689.
Elementary analysis (%):

| | | | | |
|---|---|---|---|---|
| Calculated: | C; | 60.79, | H; | 6.8 |
| Found: | C; | 60.5, | H: | 6.9 |

(5) A solution of n-butyl lithium (1.8 ml) was added dropwise to a 5 ml THF solution of 560 mg of 1,1-(ethylenedioxy)-4-methyl-3-(phenylsulfonyl)-cyclohexane and 4 mg of triphenylmethane under an argon stream at -78°C. The resulting mixture was stirred for 10 minutes and then reacted at room temperature for one hour. HMPT (1 ml) was added and the resulting mixture was cooled again to -78°C, followed by the dropwise addition of a 2 ml THF solution of 205 mg of 14-bromo-l-tetradecanol. After the reaction at - 20°C for 2 hours, the reaction mixture was poured into a saturated solution of ammonium chloride. The resulting mixture was extracted with diethyl ether. The organic layer was washed with water and saturated saline, dried over magnesium sulfate and distilled under reduced pressure to remove the solvent. The residue was purified by chromatography on a silica gel column while using hexane-ethyl acetate, whereby 1,1-(ethylenedioxy)-3-(14-hydroxytetradecyl)-4-methyl₋3-(phenylsulfonyl)-cyclohexane was obtained in the form of a colorless oil (yield: 98%). 1-1-(Ethylenedioxy)-3-(14-hydroxytetradecyl)-4-methyl-3-(phenylsulfonyl)-cyclohexane
Molecular weight: 508 (C₂₉H₄₈O₅S)
TLC: (hexane:AcOEt = 60:40) Rf=0.22
¹H-NMR (200MHz). : 1.13 (d,J=6Hz,3H,H-21), 1.28(s large, 20H, H-9a H-18), 1.43-1.6(m,9H,H-4,5,7,8,19), 1.67(m,1H,H-2), 1.89(dd, J_{gem}=12.5Hz, J=3Hz, 1H, H-6e), 2.14(t large, J=12.5Hz, 1H, H-6a), 2.43 (dd, J_{gem}= 13.8Hz, ⁴J=2.5Hz, 1H, H-2), 3.63(t,J=6.5Hz,2H,H-20), 3.83-3.97 (m, 4H, O-CH₂-CH₂-O), 7.49-7.68(m,3H,H ar.-3',4'), 7.80-7.88 (m, 2H, H ar.-2').
¹³C-NMR (50MHz) .: 16.1(C-21), 24.4(C-18), 25.6(C-5 or -7), 25.8(C-7 or -5), 29.5(C-9 to C-17), 30.3(C-8), 32.7(C-19), 34.9(C-6), 35.5(C-4), 36.2(C-2), 62.8(C-20), 63.9 and 65.1(O-CH₂-CH₂-O), 7.12(C-3), 108.4(C-1), 128.7(C ar.-3'), 130.1 (C ar.-2'), 133.3(C ar.-4'), 136.8(C ar.-1') IR(NaCl): 3510(m large, O-H), 3063(f,C-H), 2926, 2853 (f, C-H), 1585(f,C-C), 1447 (m), 1286, 1140(F, SO₂), 1096, 1083 (m,O-CH₂), 723, 693(m)
MS(Cl-NH₃): 526.4 (MNH₄, 16), 369.4 (MH₂-SO₂Ph, 28), 370.4(MH-SO₂Ph, 25), 367.3 (M-SO₂Ph, 100), 311.3(7), 307.3(8), 305.3(9), 175(17), 159.9(11), 98.9(35), 94(6), 78(11).
Elementary analysis (%):

| | | | | |
|---|---|---|---|---|
| Calculated: | C; | 67.98, | H; | 9.37 |
| Found: | C; | 67.4, | H; | 9.1 |

(6) Paratoluenesulfonic acid (20 mg) was added to a solution of 235 mg of 1,1-(ethylenedioxy)-3-(14-hydroxytetradecyl)-4-methyl-3-(phenylsulfonyl)-cyclohexane in 20 ml of chloroform and 4 ml of acetone. The resulting mixture was reacted at 50°C for 24 hours. To the reaction mixture was added 10 ml of a saturated aqueous solution of sodium bicarbonate, followed by extraction with dichloromethane. The organic layer was washed with saturated saline, dried over magnesium sulfate and distilled under reduced pressure to remove the solvent. The residue was purified by chromatography on a silica gel column while using hexane-ethyl acetate, whereby 3-(14-hydroxytetradecyl)-4-methyl-2-cyclohexen-1-one was obtained in the form of a colorless oil (yield: 75%).
3-(14-Hydroxytetradecyl)-4-methyl-2-cyclohexen-1-one
Molecular weight: 322 (C₂₁H₃₈O₂)
TLC: (hexane:AcOEt = 6:4) Rf=0.3
MS (EI): 322.2 (M⁺,37), 304.1(M-H₂O,12), 292.1(21), 164.9 (C₁₁H₁₇O,9), 151 (C₁₀H₁₅O,4), 138.1(12), 137 (C₉H₁₃O,43), 96(30), 94.9(24), 81(24), 78.9(13), 69(15), 67(25), 55(37).
Elemental analysis (%)

| | | | | |
|---|---|---|---|---|
| Calculated: | C; | 78.20, | H; | 11.88 |
| Found: | C; | 78.6, | H; | 11.9 |

### Preparation Example 2

In a similar manner to Preparation Example 1, 3-(15-hydroxypentadecyl)-4-methyl-2-cyclohexen-1-one (Compound 2) was synthesized.

### Preparation Example 3

To a methanol solution (8 ml) containing 132 mg (0.36 mmol, 1 equivalent) of 3-(12-acetoxypentadecyl)-2,4,4-trimethyl-2-cyclohexen-1-one were added 3 drops of water and 74 mg (0.54 mmol, 1.5 equivalents) of K₂CO₃. The resulting mixture was stirred at room temperature for 2.5 hours. After adjustment to pH 7 with 5% HCl, the reaction mixture was extracted with diethyl ether, dried over magnesium sulfate and distilled under reduced pressure to remove the solvent. The residue was purified by chromatography on a silica gel column, followed by elution with hexane-ethyl acetate (8:2 to 7:3), whereby 94 mg (yield: 81%) of 3-(12-hydroxydodecyl)-2,4,4-trimethyl-2-cyclohexen-1-one (Compound 3) was obtained in the form of colorless oil.
3-(12-Hydroxydodecyl)-2,4,4-trimethyl-2-cyclohexen-1-one
TLC: (hexane:AcOEr = 7:3) Rf=0.2
GC: 40 to 280°C (20°C/min) 12 min, 99%
¹H-NMR (200MHz) .: 1.13 (ds,6H,H-19,20), 1.26(s,br,16H,H-9 to H-16), 1.35-1.69(m,4H,H-8,17), 1.73(s,3H,H-21), 1.77 (t, J=7.5Hz, 2H, H-5), 2.11-2.19(m,2H,H-7), 2.43 (t, J=6.8Hz, 2H, H-6), 3.61 (t, J=6.8Hz, 2H, H-18).
¹³C-NMR (50MHz) .: 11.4(C-21), 25.7(C-16), 26.8(C-19,20), 28.8(C-8), 29.5(C-9 to C-15), 30.45(C-7), 32.7(C-17), 34.2(C-5), 36.2(C-4), 37.3(C-6), 62.9(C-18), 130.4(C-2), 165.4(C-3), 199(C-1).
IR.: 3440 (broad OH), 2925, 2852(w,C-H), 1666(w,C=O), 1605(s,C=C), 1467 (s,C-H).

### Preparation Example 4

In a similar manner to Preparation Example 3, the below-described compounds were obtained. The numeral in parentheses indicates the Rf value of TLC with a 7:3 mixed eluent of hexane and ethyl acetate.
(1) 3-(15-Hydroxypentadecyl)-2,4,4-trimethyl-2-cyclohexen-1-one (Compound 4) (Rf=0.29)
(2) 3-(18-Hydroxypentadecyl)-2,4,4-trimethyl-2-cyclohexen-1-one (Compound 5) (Rf=0.25)

### Test 1

Nerve stem cells were prepared from murine brain in accordance with the method of Weiss and Reynolds (1996). Specifically, striatum was dissected from mouse embryo. The cells were dispersed in a culture solution containing EGF (20 ng/ml) and incubated at 37°C under 5% CO₂ for 5 days, followed by centrifugation in "Dissociation Medium" (product of Sigma) at 400 rpm for 5 minutes, whereby neurospheres, that is, nerve stem cell clusters, were obtained. The resulting neurospheres were dispersed in a culture solution and incubated under the same conditions to yield secondary neurospheres.

Sterile glass coverslips in 24-well plates were treated overnight with a solution of polyornithin (30 µg/mL) and then rinsed three times in a phosphate buffer. The neurospheres were inoculated to give 20 to 50 neurospheres per glass coverslip. Compounds 1 to 5 obtained in Preparation Examples 1 to 4 and adjusted with ethanol to have a concentration of 10⁻⁶M were added and the mixtures were incubated until neurospheres differentiated sufficiently (typically, 24 hours).

The neurospheres which differentiated sufficiently were fixed in 4% para-formaldehyde, rinsed in a phosphate buffer, added with 0.5% Triton-X100 for 5 minutes and then, rinsed again in a phosphate buffer. Mouse monoclonal antibodies anti-MAP2 (2a + 2b) (product of Sigma) for identifying neurocytes, mouse monoclonal antibodies anti-04 (product of Boeringher) for identifying oligodendrocytes, and rabbit polyclonal anti-GFAP (DAKO) for identifying astrocytes were added, followed by incubation at room temperature for 1 hour or overnight at 4°C. After the addition of anti-mouse IgM antibodies and a fluorescence indicator, the mixture was incubated at room temperature for 1 hour and then rinsed in a phosphate buffer. The glass coverslip was placed on a confocal microscope, through which differentiation of neurospheres was observed.

As a result, Compounds 1 to 5 were found to have an action for promoting differentiation of neurospheres, that is, nerve stem cell clusters, into neurocytes.

**Table 1**

| | Neurocytes | Oligodendrocytes | Astrocytes |
|---|---|---|---|
| Compound 1 | ↑ | ↑ | ↓ |
| Compound 2 | ↑ | ↑ | ↓ |
| Compound 3 | ↑ | → | ↓ |
| Compound 4 | ↑ | ↑ | ↓ |
| Compound 5 | ↑ | → | ↓ |

### Industrial Applicability

A medicament comprising the cyclohexenone long-chain alcoholic derivative represented by the formula(I) is useful as a preventive or remedial drug for diseases caused by degeneration or reduction of various tissues or cells, or by cell death, for example, nervous diseases such as Alzheimer's disease, Pick's disease, Parkinson disease, Huntington chorea, spino-cerebellar degeneration and amyotrophic lateral sclerosis; bone diseases such as osteoporosis and fracture; circulatory diseases such as angina pectoris, retinopathy, arteritis obliterans, and myopathy such as muscular dystrophy and congenital myopathy.

## Claims

1. Use of a cyclohexenone long-chain alcoholic derivative represented by the following formula (1): 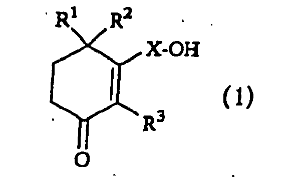 [wherein, R¹, R² and R³ each independently represents a hydrogen atom or a methyl group and X represents a linear or branched C₁₀₋₂₈ alkylene or alkenylene group] or a pharmaceutically acceptable salt, solvate or hydrate thereof for promoting differentiation-induction of a nerve stem cell into a neurocyte, whereby methods of treating the human or animal body by surgery or therapy and diagnostic methods practiced on the human or animal body are excluded.

## Patentansprüche

1. Verwendung eines Cyclohexenon-Derivats mit langer alkoholischer Kette, das dargestellt wird durch die folgende Formal (1): 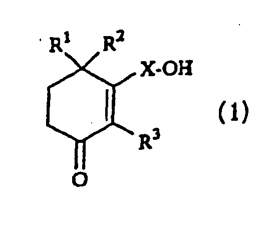 [wobei R¹, R² und R³ jeweils unabhängig voneinander für ein Wasserstoffatom oder eine Methylgruppe stehen und X für eine lineare oder verzweigte C₁₀₋₂₈ Alkylen- oder Alkenylengruppe steht] oder ein pharmazeutisch unbedenkliches Salz, Solvat oder Hydrat davon zur Förderung der Induktion einer Differenzierung von einer Nervenstammzelle zu einem Neurozyten, außer Verfahren zur chirurgische oder therapeutischen Behandlung des menschlichen oder tierischen Körpers sowie Diagnostizierverfahren, die am menschlichen oder tierischen Körper vorgenommen werden.

## Revendications

1. Utilisation d'un dérivé alcoolique à longue chaine de cyclohexènone, représenté par la formule suivante (1) : 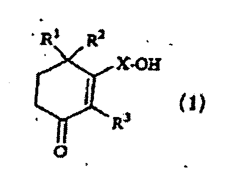 [dans laquelle R¹, R² et R³ représentent chacun indépendamment un atome d'hydrogène ou un groupe méthyle et X représente un groupe alkylène ou alcénylène linéaire ou ramifié et en C₁₀₋₂₈] ou d'un sel, solvate ou hydrate pharmaceutiquement acceptable de celui-ci, pour favoriser la différenciation-induction d'une cellule nerveuse souche en un neurocyte, les méthodes de traitement du corps humain ou animal par chirurgie ou thérapie et les méthodes de diagnostic pratiquées sur le corps humain ou animal étant exclues.
